# EUROPEAN PATENT APPLICATION

(11) **EP 4 289 376 A1**
(43) Date of publication of application: **13.12.2023**
(21) Application number: 22178447.3
(22) Date of filing: 10.06.2022
(51) Int. Cl.: A61B 17/34

(54) **NEEDLE GUIDE**

(71) Applicant: AtriAN Medical Limited, H91 E79C Galway (IE)
(72) Inventor: DEANE, Stuart, Upper Newcastle, Galway, H91 E79C (IE); O'BRIEN, Barry, Upper Newcastle, Galway, H91 E79C (IE); REILLY, John, Upper Newcastle, Galway, H91 E79C (IE); COFFEY, Kenneth, Upper Newcastle, Galway, H91 E79C (IE)
(74) Representative: Murgitroyd & Company

(57) **Abstract**

A needle guide comprising a plurality of indication marks to assist in conveying trajectories to a user, to assist in the insertion of a needle or the like device, into a patient or model for training purposes.

## Description

The present invention relates to a needle guide, and methods of using, in particular, to a needle guide for the insertion of a needle into the pericardial space and onto the surface of the heart of a patient by use of the subxiphoid access.

### Background

Inserting needles, cannula and the like, into a patient for various purposes including vaccines, exploratory investigations or insertion of medical devices are now commonplace procedures. Getting this correct is still important with the consequences of wrongful insertion causing pain or tissue damage to a patient and ultimately even proving fatal. Various guiding procedures or devices are known, each with its own usefulness and limitations.

Fluoroscopy guided needle access uses ionizing radiation directed to the area of the patient and can show, in real time, the tissue of the patient and travel of the needle being inserted.

A known alternative to using radiation, for needle guided access, is the use of ultrasound. Ultrasound guided needle access typically requires a high level of user experience to be effective and is not effective at multiple planes or layers of tissue. In use ultrasound guided imaging often requires frequent resetting.

For treating or investigating some heart conditions interventional devices may be positioned into the pericardial space and onto the surface of the heart by use of subxiphoid access. A subxiphoid access is carried out by guiding a needle through the skin, under the subxiphoid process and placing the tip of the needle adjacent to the anterior surface of the heart. Experienced clinicians have an intrinsic knowledge of the needle access site and the appropriate angle to navigate the needle to the correct location. There are risks of wrongful insertion with serious consequences and these risks are greater with less experienced surgeons.

The present invention aims to address some of the problems of the prior art, for example, prior art methods of guiding a needle for, or during insertion into a patient.

### Summary

According to an aspect of the present invention, there is provided a needle guide, for positioning of a needle or the like, wherein the needle guide comprises:
- a guide body, wherein the guide body comprises: a lower contact surface and an upper surface wherein the lower contact surface is configured for positioning, on a patient, at least partially over the xiphoid process of the patient;
- a port;
- a first indication mark configured to be positioned over an inferior portion of the xiphoid process, with an orientation that the port of the needle guide is in an inferior position to the first indication mark, such that when in use, in this position and orientation, the first indication mark indicates the position of the inferior portion of the xiphoid process of the patient and the port of the needle guide is inferior in position to the first indication mark; and,
- a second indication mark that is, or at, or on, or conveys or projects, or indicates, or is configured to indicate, a trajectory of: 217 degrees; or 217 degrees plus or minus 11 degrees; or between 206 degrees and 228 degrees; from the median line, on the coronal plane, through or to, the port, when measured starting in the sinister direction from the median line.

Advantageously the present invention may assist a user to insert a needle, or similar device, into a patient to the correct or desired location. The present invention may assist a user to insert a needle, or similar device, into a patient through the skin at the correct or desired location on the skin, to enable the needle to reach the correct or desired final location within the body of the patient. For example, the present invention may assist for anterior access to the heart. The present invention may assist for sub xiphoid process access to the heart. In alternative embodiments, the present invention may assist in inferior access to the heart. The present invention greatly reduces risks of inserting a needle, or similar device, into the wrong, or unintended location within the body. The present invention may greatly reduce the risk of causing needless pain or harm, or even death, to the patient. The port of the present invention may be a guide to the desire location of, where it may be less risk, to insert the needle through the skin, than other areas of the skin, and other routes through the body. This may be especially so when the needle guide of the present invention is positioned on the patient at the correct or desired location. The first indication mark may act to convey or indicate the angle of insertion, or at least one dimension of an angle of insertion, of a needle or similar device, that may be less risk to the patient than other angles of inserting a needle, in particular at this location, when starting insertion of the needle from the desired starting point indicated by the invention. For example, at the location of the port of the device when positioned on the patient in the correct, or desired, position.

In some embodiments the second indication mark is, or at, or on, or conveys or projects, or indicates, or is configured to indicate, a trajectory of: 217 degrees plus or minus 6 degrees; or between 210 degrees and 222 degrees; or is between 215 degrees and 220 degrees, from the median line, on the coronal plane, through or to, the port, when measured starting in the sinister direction from the median line.

In some embodiments, the first indication mark is configured to be positioned over the most inferior portion of the xiphoid process, with an orientation that the port of the needle guide is in an inferior position to the first indication mark, such that when in use, in this position and orientation, the first indication mark indicates the position of the most inferior portion of the xiphoid process of the patient and the port of the needle guide is inferior in position to the first indication mark. Aligning the first indication mark to the most inferior portion of the xiphoid process of the patient is a relative easy task to perform for the skilled user. The skilled user is likely to be easily able to determine the location of the most inferior portion of the xiphoid process from looking at the patient, but in some patients from feeling the anterior, location, of the xiphoid process, through the skin of the patient. Viewing and if needed feeling the anterior portion of the inferior portion of the ribs may also assist, the xiphoid process is located inferior on the sternum where the inferior portion of the two anterior halves of the rib cage meet.

Once the most inferior portion of the xiphoid process is located the first indication mark may be position over the most inferior portion of the xiphoid process to easily indicate the position of the most inferior portion of the xiphoid process. This advantageously sets the first location marker for the invention, the needle guide.

In particular embodiments, of the present invention, when the needle guide is positioned on the body, over the xiphoid process of a patient, such that the first indication mark corresponds in position with the xiphoid process, or corresponds with the lower edge of the rib cage indicating the position of the xiphoid process, and the orientation of the needle guide is such that the port of the needle guide is in the inferior direction; then the port should be in a position that offers potentially greater certainty, or chance, for inserting a needle to the anterior surface of the heart than some other regions of the skin.

In some embodiments, the first indication mark comprises an edge of the guide body of the needle guide. In use the first indication mark would be positioned over the inferior portion, or over the most inferior portion, of the xiphoid process. In use the first indication mark would be positioned over the inferior portion, or over the most inferior portion, of the xiphoid process and configured and orientated such that the port of the guide body would be inferior in position to the first indication mark.

The skilled person would understand that the determining of the location of the inferior, or most inferior portion of the xiphoid process would be exact enough for working the invention.

In some embodiments, the first indication mark comprises, at least one arm that extends from the guide body in the same plane as the guide body. Having the first indication mark comprise an arm that extends from the guide body in the same plane is a simple means to have the first indication mark. This simple construction of the first orientation mark is easy to see and align with the xiphoid process and potentially easy and efficient to manufacture.

In particular embodiments, the first indication mark comprises two arms that extend from the guide body in the same plane as the guide body. In particular embodiments, each of the two arms of the first indication mark extend from the guide body in the same plane as the guide body. Having a first indication mark that comprises two arms may assist in easier positioning of the needle guide on the body of a patient in the desired position than one arm, for example, to position the needle guide over the xiphoid process of a patient and in relation to the rib cage.

In particular embodiments the two arms of the first indication mark extend perpendicularly from the guide body in the same plane as the guide body. In alternative embodiments the two arms of the first indication mark extend non-perpendicularly from the guide body in the same plane as the guide body.

In addition, when the needle guide of the present invention is positioned on the body, over the xiphoid process of a patient, such that the first indication mark corresponds in position with the xiphoid process, for example, the most inferior portion of the xiphoid process, and the orientation of the needle guide is such that the port of the needle guide is in the inferior direction; then a second indication mark may convey the angle of inserting a needle through the skin area identified or exposed by the port, that offers potentially greater certainty, or chance, of inserting a needle to the heart, for example the anterior surface of the heart, than from other areas of the body or by inserting at other angles.

In some embodiments, the second indication mark comprises an arm that extends from the guide body in the same plane as the guide body. Having the second indication mark comprise an arm that extends from the guide body in the same plane, as the guide body, is a simple means to have the second indication mark. This simple construction of the second orientation mark is potentially easy to manufacture and potentially cheap to manufacture.

When the needle guide is positioned on a patient at the desired location, for example, at least partly over the xiphoid process, the second indication mark may indicate an angle, or at least one dimension of an angle, towards the heart, through the port of the needle guide, that by inserting a needle along this angle has a greater chance of reaching the heart, and in particular the pericardial space of the heart of a patient, or anterior surface of the heart.

In some embodiments, the second indication mark comprises a triangular shape. In some embodiments, the second indication mark comprises an isosceles triangle shape. Advantageously the triangle shape can help point or assist the user to the desired direction and orientation to align the needle.

In particular embodiments, the triangular shape of the second indication mark is configured to point to the port of the needle guide. In particular embodiments, the second indication mark comprises an isosceles triangle in shape wherein the two equal angles of the isosceles triangle are further in distance from the port of the needle guide than the other angle, different from the two equal angles of the isosceles triangle. In particular embodiments, wherein the second indication mark comprises an isosceles triangle in shape, a first edge of the second indication mark extends from the guide body at 206 degrees from the centre of port of the needle guide measured, first moving sinisterly from the median line, on same plane as the guide body. In particular embodiments, wherein the second indication mark comprises in shape an isosceles triangle, a second edge of the second indication mark extends from the guide body at 228 degrees from the centre of port of the needle guide measured, first moving sinisterly from the median line, on the same plane as the guide body. Advantageously wherein the second indication mark is triangular in shape and orientation such that it is pointing to the port and preferably to the centre of the port, this assists the user to align the needle to the port and preferably to the centre of the port. In embodiments wherein the outer edges of the triangular shaped second indication mark are 206 and 228 degrees from the centre of the port measured from the median line, allows a user to easily align a needle for the desired angle for insertion into the patient.

In some particular embodiments, wherein the second indication mark comprises in shape an isosceles triangle, the second indication mark may further comprises a line on the upper surface, wherein the line, at least partially, extends from the guide body at 217 degrees; or 217 degrees plus or minus 11 degrees; or between 210 and 225 degrees, from the centre of port of the needle guide measured, first moving sinisterly from the median line, on the same plane as the guide body. Advantageously having the second indication mark comprising a visible line facing upwards in use, at 217 degrees from the centre of the port allows a user to easily align the needle at this same angle pointing towards the centre of the port and thus a greater chance to reach the anterior surface of the heart safely.

In some embodiments, the port is circular. Having a port that is circular may offer a shape, which is easy to manufacture. The circular shape may also avoid corners or shape edges to harm the patient, or catch the needle, or other items. The circular shape may offer a good balance of the desired area to insert the needle through the skin with good strength of the structure of the needle guide. In alternative embodiments the port is a different shape, and does not necessarily need to be circular in shape.

In some embodiments, the second indication mark indicates a trajectory that goes through, or to, the port, for example, the centre of the port. In some embodiments, the second indication mark is at an angle from the needle guide that indicates a trajectory that goes through, or to, the port, for example, the centre of the port. The second indication mark may extend from the main body of the needle guide at an angle that indicates a trajectory that goes through, or to, the port, for example the centre of the port. The second indication mark as described is an easy means to indicate an angle of inserting a needle that may have a greater chance of being inserted to the anterior surface of the heart, for example, the pericardial space of the heart of the patient, than other angles, especially at other positions on the body.

In some embodiments, the needle guide further comprises a third indication mark. In some embodiments, the third indication mark comprises an arm extending from the guide body.

In some embodiments, the third indication mark comprises an arm extending, at least partially perpendicular, from the guide body. In some embodiments, the third indication mark comprises an arm extending, at least partially, perpendicular from the upper surface of the guide body. The third indication mark arm is capable of being orientated perpendicular from the upper surface of the needle guide body, away in direction from the body of the patient, when in use. Having a third indication mark orientated perpendicular from the upper surface of the needle guide body enables another dimension of a trajectory, to be indicated. Thus, the third indication mark allows a greater chance that by inserting a needle along the trajectory indicated by the third indication mark that the needle may reach the desired location, for example, the anterior surface of the heart, or pericardial space of the heart of a patient.

In particular embodiments, the third indication mark is, or at, or on, or conveys or projects, or indicates, or is configured to indicate, a trajectory of: 34 degrees; or 34 degrees plus or minus 12 degrees; or between 22 degrees and 46 degrees; from the coronal plane in an anterior direction, through, or to, the port. In particular embodiments, the third indication mark indicates a trajectory of: 34 degrees; or 34 degrees plus or minus 12 degrees; or between 22 degrees and 46 degrees; from the coronal plane, in the anterior direction through, or to, the port, when the needle guide is orientated in the orientation for use. In particular embodiments, the third indication mark is, or at, or on, or conveys or projects, or indicates, or is configured to indicate, a trajectory of: 34 degrees; or 34 degrees plus or minus 12 degrees; or between 22 degrees and 46 degrees; from the horizontal plane, in an anterior direction, through, or to, the port, when in an orientation for use on a patient. In particular embodiments, the third indication mark is, or at, or on, or conveys or projects, or indicates, or is configured to indicate, a trajectory of: 34 degrees; or 34 degrees plus or minus 12 degrees; or between 22 degrees and 46 degrees; from the horizontal plane in an anterior direction, is indicated, through, or to, the port, when in an orientation for use on a patient. The third indication mark that indicates a trajectory along the trajectory of these angles and direction gives a greater chance that by inserting a needle along this trajectory may reach the desired location, for example, the anterior surface of the heart, or pericardial space of the heart of a patient. In use the user can easily align the needle with the trajectory conveyed by the third indication mark to assist in insertion of the needle in the desired location.

In particular embodiments, the third indication mark comprises an arm wherein the arm, of the third indication mark, is configured at least partially in a trajectory of: 34 degrees; or 34 degrees plus or minus 12 degrees; or between 22 degrees and 46 degrees; from the coronal plane in an anterior direction through, or to, the port. In particular embodiments, the arm of the third indication mark is configured at least partially in a trajectory of: 34 degrees; or 34 degrees plus or minus 12 degrees; or between 22 degrees and 46 degrees; from the coronal plane, in the anterior direction through, or to, the port, when orientation in the orientation for use. In particular embodiments, the arm of the third indication mark is configured at least partially in a trajectory of: 34 degrees; or 34 degrees plus or minus 12 degrees; or between 22 degrees and 46 degrees; from the horizontal plane, in the anterior direction, through, or to, the port, when in an orientation for use on a patient. The arm of third indication mark therefore indicates a trajectory along the trajectory of these angles and gives a greater chance that by inserting a needle along this trajectory may reach the desired location, for example, the anterior surface of the heart, or pericardial space of the heart of a patient. In use, the user can easily align the needle with the trajectory conveyed by the third indication mark to assist in insertion of the needle in the desired location.

The arm of the third indication mark may comprise an elongated guide body. The elongated guide body may be configured to extend at least partially along the, or one of the, desired trajectory. By this configuration the third arm may easily convey the desire trajectory.

In some embodiments, the third indication mark indicates a trajectory through, or to, the centre of the port. The third indication mark that indicates a trajectory through, or to, the centre of the port may give a greater chance that by inserting a needle along this trajectory, or may reach the desired location, for example, the heart; or anterior surface of the heart; or pericardial space of the heart of a patient, than a different trajectory, especially when the needle guide is positioned over the xiphoid process.

In some embodiments the arm of third indication mark comprises a distal edge configured that projects, or indicates, a trajectory of: 34 degrees; or 34 plus or minus 12 degrees; or is between 22 degrees to 46 degrees, or is between 24 degrees and 40 degrees; or is between 30 degrees and 39 degrees; or is between 20 degrees and 50 degrees, from the coronal plane, in the anterior direction, through, or to, the port. Having the arm of the third indication mark comprise the distal edge that is configured and corresponds to the desire angle to project for the trajectory of the inserting needle is an easy to use alignment tool. A user can easily see the outer edge of the third indication mark to align the needle for inserting into the patient at the desired angle and orientation. The edge of the third indication mark makes seeing the desired angle of the desired trajectory; and consequently aligning the needle to that desired trajectory, easy. Such a design is user friendly. Such a design for the third indication mark is easy to manufacture.

In some embodiments the arm of third indication mark comprises a distal edge configured that is, or at, or on, or conveys or projects, or indicates, or is configured to indicate, a trajectory of: 33 degrees; or 33 plus or minus 12 degrees; or is between 21 degrees to 45 degrees, from the coronal plane, in the anterior direction, through, or to, the port. Having an arm of the third indication mark comprise the distal edge that is configured and corresponds to one degree less than the desire angle to project for the trajectory of the inserting needle is an easy to use alignment tool, wherein a user may contact the arm of the third indication mark to align the needle to the desired trajectory, that will have a greater chance of reaching the desired location upon insertion. A user can easily see, and contact, the outer edge of the third indication mark to align the needle for inserting into the patient at the desired angle and orientation. The edge of the third indication mark makes seeing, and positioning, the desired angle of the desired trajectory; and consequently aligning the needle to that desired trajectory, easy. Such a design is user friendly. Such a design for the third indication mark is easy also to manufacture.

In particular embodiments, the third indication mark comprises a line. In some embodiments the third indication mark comprises a visible line configured to at least partially be, or in, or of, or convey, the trajectory of: 34 degrees; or 34 degrees plus or minus 12 degrees; or between 22 degrees and 46 degrees, from the coronal plane, in the anterior direction, through, or to, the port. In some embodiments the third indication mark comprises a visible line configured to at least partially be, or in , or of, or convey, the trajectory of: 34 degrees; or 34 degrees plus or minus 12 degrees; or between 22 degrees and 46 degrees, from the coronal plane, in the anterior direction, through, or to, the port, when in an orientation for use. In particular embodiments, the third indication mark comprises a visible line that corresponds to the desired trajectory. Where the third indication mark comprises a line this is an easy to use third indication mark, easy for a user to align a needle for inserting into a patient with the line of the third indication marker. In embodiments where the third indication marker comprises a line, this is a simple to manufacture type of indication mark.

In particular embodiments, the third indication mark comprises a tubular shape. Where the third indication mark comprises a tubular shape this may allow easy positioning of a needle, for inserting into a patient, within a range of orientations and trajectories that may give a greater chance of reaching the desired location, for example, the anterior surface of the heart or the pericardial space of the heart, of a patient. In some embodiments, the inner side walls of the tubular shape, of the third indication mark, may form the boundaries of the desired orientation and trajectories that are suitable. For example, for a greater chance of an inserting needle following these trajectories though the port of the needle guide, reaching the desire location, for example, the heart or pericardial space of the heart, when the needle guide is positioned in the desire location and orientation. In particular embodiments, a portion of the inner side walls are configured to, at least partially be, or in, or of, or convey, the trajectory of: 22 degrees, from the coronal plane, in the anterior direction, through, or to, the port, when in an orientation for use. In particular embodiments, a portion of the inner side walls are configured to, at least partially be, or in, or of, or convey, the trajectory of: 46 degrees, from the coronal plane, in the anterior direction, through, or to, the port, when in an orientation for use. In particular embodiments, the third indication mark comprises a tubular shape wherein the lumen of the tubular shaped third indication mark is configured to at least partially be, or in, or of, or convey, the trajectory of: 34 degrees; or 34 degrees plus or minus 12 degrees; or between 22 degrees and 46 degrees, from the coronal plane, in the anterior direction, through, or to, the port. Advantageously a user can have confidence that once the needle is inside the tubular shaped third indication mark, when the device is positioned correctly, that inserting the needle along the trajectory indicated by the tubular shaped third indication mark, will be a desired trajectory. Having a tubular shaped third indication marks configured with side walls at the outer limits of the desired trajectories means that the device is very easy to use for aligning the needle to the desired trajectory or a trajectory that has a greater chance for an inserting needle to arrive at the desired location. In practice the needle for inserting into the patient or model maybe positioned in contact with inner side walls of the tubular shaped third indication mark.

In particular embodiments, the third indication mark comprises a frustoconical shape. In particular embodiments the third indication mark comprises a hollow frustoconical shape wherein further comprises a configuration comprising a smaller diameter opening nearer to the port than the larger diameter opening, when in normal orientation for use. Where the third indication mark comprises a frustoconical shape, especially a hollow frustoconical shape, this may allow easy positioning of a needle, for inserting into a patient, within a range of orientations and trajectories that may give a greater chance of reaching the desired location, for example, the anterior surface of the heart or the pericardial space of the heart, of a patient. The inner side walls of the frustoconical shape may form the boundaries of the desired orientation and trajectories that are suitable, for a greater chance of an inserting needle following these trajectories though the port of the needle guide, reaching the desire location, for example the heart; or anterior surface of the heart; or pericardial space of the heart, when the needle guide is positioned in the desire location and orientation. If the frustoconical shape is orientated that it is pointing towards the port of the needle guide, that the smaller opening is nearer to the port, this assists in defining the suitable trajectories that may be suitable to reach the desire location, by inserting a needle along these trajectories, when the needle guide is positioned in the desired location and orientation. A hollow frustoconical shape is a subset of a tubular shape and therefore the possible trajectories mentioned above relating to the tubular shaped third indication mark also apply equally to a frustoconical shaped third indication mark.

In some embodiment, the needle guide is configured that the port is between 5 millimetres and 15 millimetres in the inferior direction of the first indication mark of the needle guide when in normal use. In some embodiment, the needle guide is configured that the port is between 5 millimetres and 15 millimetres in the inferior direction of the first indication mark of the needle guide along the median line or longitudinal axis when orientated for normal use. In some embodiment, the needle guide is configured that the port is between 5 millimetres and 15 millimetres in the inferior direction from the most inferior point of first indication mark of the needle guide when in normal use. In particular embodiments, the needle guide is configured that the port is between: 3 millimetres and 17 millimetres; or 4 millimetres and 16 millimetres; or 6 millimetres and 14 millimetres; or 7 millimetres and 13 millimetres; or 8 millimetres and 12 millimetres, in the inferior direction of the first indication mark of the needle guide when in normal use. In some embodiments, the port is configured 10 millimetres in the inferior direction from the first indication mark of the needle guide, when in normal use. In these embodiments, the distance from the first indication mark to the port may be measured from the most inferior point of the first indication mark on the longitudinal axis to the port, for example the most superior point of the port on the longitudinal axis.

In some embodiments, the needle guide is configured such that the centre of the port is 10 millimetres in the inferior direction from the first indication mark of the needle guide, when in normal use. In some embodiments, the needle guide is configured such that the centre of the port is 10 millimetres in the inferior direction from the most inferior point of first indication mark of the needle guide, when in normal use. In some embodiments, the needle guide is configured such that the centre of the port is: 8 millimetres; or 9 millimetres; or 11 millimetres; of 12 millimetres, in the inferior direction from the first indication mark of the needle guide, when in normal use. In some embodiment, the centre of the port is configured to be in the range of 5 to 15 millimetres in the inferior direction from the first indication mark of the needle guide when in normal use. In particular embodiments, the needle guide is configured such that the centre of the port is: between: 3 millimetres and 17 millimetres; or 4 millimetres and 16 millimetres; or 6 millimetres and 14 millimetres; or 7 millimetres and 13 millimetres; or 8 millimetres and 12 millimetres, in the inferior direction of the first indication mark of the needle guide when in normal use. The distance from the first indication mark to the centre of the port may be measured from the most inferior point of the first indication mark to the centre of the port. It has been found that despite different sizes of patients this spacing may be suitable to the vast majority of patients, in order to insert a needle or the like through the skin to reach the desired location, for example, the anterior surface of the heart, or pericardial space of the heart, of a patient. Especially when the needle is inserted at an appropriate angle and orientation.

In some embodiments, the needle guide is configured such that the centre of the port is on the longitudinal axis of the needle guide. Advantageous this allows the centre of the port to be on the median line of the patient, or model when the needle guide is orientated for normal use.

In some embodiments, the needle guide comprises a fourth indication mark comprising a plurality of marks spaced apart from each other by a defined distance. In some embodiments, the needle guide comprises a fourth indication mark comprising a plurality of marks spaced apart from each other by a defined distance. In some embodiments, the needle guide comprises a fourth indication mark comprises a plurality of marks configured wherein each mark of the plurality of marks is a defined distance from the port. In embodiments wherein a fourth indication mark is configured to indicate at least a distance from the port, for example, the centre of the port along a trajectory, this allows a user to easily estimate a distance of travel of a needle being inserted. In embodiments having an indication mark that indicates distance, may enable a user to know how far a needle has been inserted into a patient by the distance travelled along the indication mark that indicates distance.

In some embodiments the fourth indication mark is configured to convey or indicate a defined distance from the port, or centre of the port, along a trajectory, for example 50 millimetres. In particular embodiments the fourth indication mark comprises a plurality of marks each configured to convey or indicate different defined distances from the port, or the centre of the port.

In some embodiments, the third indication mark comprises the fourth indication mark; or the fourth indication mark comprises the third indication mark. The third and fourth indication mark may comprise a mark or marks that indicate distance along a trajectory, for example, rows of lines spaced apart by 1 millimetres. In other embodiments, the rows of marks may be spaced apart form the next mark by different distances, for example, by 2 millimetres, or 5 millimetres.

In some embodiments, the second indication mark and third indication mark are the same, or part of the same element. In some embodiments the second indication mark comprises an arm that is, or is on, or conveys a trajectory of an angle that is: 217 degrees; or 217 degrees plus or minus 11 degrees; or is between 206 degrees to 228 degrees, from the Median line of the patient, or zero degrees position on the coronal plane, through, or to, the port; and the third indication mark comprises an arm that is, or is on, or conveys, or indicates, or projects, a trajectory of: 34 degrees; or 34 degrees plus or minus 12 degrees; or is between 22 degrees to 46 degrees, from the Coronal plane, in the anterior direction, through, or to, the port, wherein the first indication mark comprises the second indication mark. In some embodiments, one indication mark may convey both parameters of the second and third indication marks. In some alternative embodiments, one indication mark may convey parameters of the second and third and fourth indication marks.

In some embodiments the second indication mark, or the third indication mark, or both the second indication mark and third indication mark comprises a slot. The slot may be suitable for entry, or exit, of a needle, to or from the lumen of a tubular shaped indication mark, for example. In some embodiments, the second or third indication comprises a tubular shape comprising a slot in the longitudinal direction of the wall of the tubular shape. In some embodiments, the second or third, or both, indication mark comprising a hollow frustoconical shape comprising a slot in the longitudinal direction of the wall of the hollow frustoconical shape. The slot in the second indication mark, or third indication mark, or both, may allow easy access of the needle by a user into the lumen, or enclosed area of the hollow frustoconical shape, that projects the desired trajectories for inserting the needle through the skin to have a greater chance of reaching the desired location, for example, the anterior surface of the heart, or pericardial space of the heart, of a patient. The slot, for example, in the second or third indication mark, may allow easier removal of the needle guide, while leaving the needle in place, for example the desired trajectory. This is advantageous as it enables easy removal of the needle guide, to allow easier movement of the needle, after the positioning of the needle has been completed. Likewise, if the indication mark comprises a tubular shape or hollow frustoconical shape having the slot in the wall of the tubular, or frustoconical, wall, this may allow easy access of the needle by a user into the enclosed area that projects the desired trajectories for inserting the needle through the skin to have a greater chance of reaching the desired location, for example, the heart; the anterior surface of the heart; or pericardial space of the heart, of a patient. The user may insert the needle into the enclosed area of the second or third indication mark; or the tubular or frustoconical shaped structure of the indication mark, wherein inserting a needle through the skin, for example, within the port area, along the trajectories within the enclosed area, have a greater chance of reaching the desired location, for example, the heart; the anterior surface of the heart; or pericardial space of the heart, of a patient.

In some embodiments the second and third indication marks comprise a tubular or frustoconical shape. In some embodiments, where there is an indication mark comprising a tubular or frustoconical shape, there may further comprise an extension element configured to position the tubular or frustoconical shaped indication mark. In some embodiments the extension element is rigid.

In some embodiments the slot opens from the larger opening of the funnel or frustoconical shaped indication mark. In other embodiments the slot opens from the smaller opening of the funnel or frustoconical shaped indication mark.

In some embodiments the slot opens from the opening of the opening of the tubular or funnel or frustoconical shaped indication mark, that is nearest to the guide body. In some embodiments the slot opens from the opening of the opening of the tubular or funnel or frustoconical shaped indication mark, that is furthest from the guide body.

In some embodiments, the needle guide comprises adhesive. In some embodiments, the contact surface of the needle guide comprises adhesive. Having adhesive on the contact surface of the needle guide, that will contact the patient may allow easy, temporary, fixing of the needle guide in position. The adhesive is also such that the guide can be easily removed.

In some embodiments the third indication mark comprises an arm that is on the same plane as the guide body and the third indication mark further comprises a hinge portion configured to enable a portion of the arm of the third indication mark to be orientated at least partially perpendicular from the guide body. In some embodiments, the third indication mark comprises a hinged portion and further comprises wherein the arm of the third indication mark is configured to be articulated from a configuration where the arm of the third indication mark is planar with the guide body, to a second configuration wherein the arm of the third indication mark is at least partially perpendicular to the guide body in the anterior direction. Ideally, in the second configuration the arm is in the desired orientation to convey the desires angle from the coronal plane in the anterior direction. In embodiments where the third indication mark may be initially in a flat configuration, this advantageous may make manufacturing, transport and storage more efficient.

In some embodiments, the guide body is rectangular in shape. Advantageously when the guide body is rectangular an edge of the guide body may be the first indication mark and thus it may be easy to align the first indication mark over the xiphoid process. In alternative embodiments, the guide body may comprise alternative shapes.

In some embodiments, the guide body has a length between: 10 and 80 millimetres; or 20 and 70 millimetres; or 25 and 75 millimetres; or 30 and 50 millimetres; or 35 and 45 millimetres.

In some embodiments, the guide body has a width between: 5 and 50 millimetres; or 5 and 40 millimetres; or 5 and 30 millimetres; or 5 and 25 millimetres; or 15 and 25 millimetres.

In use, the user of a needle guide according to the present invention, may position the contact surface of the guide body over the xiphoid process of a patient or model. Ideally, the guide body will be positioned over the inferior portion of the xiphoid process, orientated such that the port of the needle guide is in the inferior direction from the guide body. Ideally, the port will be then positioned approximately 10 millimetres from the xiphoid process of the patient or model. Ideally, the contact surface, in some embodiments, may comprise adhesive to assist in holding the guide body on the patient. The exact positioning of the guide body over the xiphoid process of the patient or model should be good enough for purpose that the port is approximately 10 millimetres in the inferior direction from the xiphoid process. In some method of using this may comprise positioning the first indication mark over the lower portion of the xiphoid process or indeed over the most inferior portion of the xiphoid process. When positioned as described herein the second indication mark will be located on, or will convey an angle of a trajectory through to or to port and this will be in the direction of the heart of the patient or model. By this assistance alone, the invention is assisting a user to optimise trajectories to reach the heart. With the use of third indication mark another dimension of the optimised or desired trajectory is given - the angle from the coronal plane towards the heart. When a user aligns the needle with the second and third indication marks, or the trajectories indicated by the second and third indication marks, through or at least to the port, this helps optimise the appropriate or desired trajectories to reach the heart, for example, the anterior surface of the heart. The second and third indication marks give or indicate, two dimensions of trajectories, which by aligning with, or at least aligning within the ranges of, optimising safe trajectories for the insertion of the needle. In some embodiments that an indication mark comprises a visual line or row of marks or the like, to convey a trajectory, the user visually aligns the needle with the line or other mark of the indication mark. By aligning the projected trajectories from one or more indication marks may optimise the desired trajectory to safely reach the heart, or anterior surface of the heart etc. In some embodiments that the needle guide is manufactured in a single plane, but has a different configuration for use, the user should pivot an indication mark for example the third indication mark to a second configuration that then that indication mark conveys the desired trajectory. In some embodiments, one or more indication marks together comprise a hollow, tubular or frustoconical shape, in which the user may position the needle. The trajectories conveyed from the inside of these hollow structures may be optimised to be the desired trajectories for inserting the needle. One or more of the indication marks of the present invention may comprise marks that indicate distance along a trajectory and therefore the user may align the needle and note distance moved from one mark to another along the trajectory. In some embodiments comprising a slot, after a user has positioned the needle using the needle guide, the needle can be held in position pointing in the desired direction and the needle guide removed assisted by having the slot so as not to touch the needle when removing the needle guide.

According to another aspect of the present invention there is provided a method of guiding a needle for insertion into the pericardial space of the heart of a patient.

According to another aspect of the present invention there is provided a method of guiding a needle for insertion to the heart, for example, to the anterior surface of the heart of a patient.

In some embodiments, a method of guiding a needle, comprising the steps of:
- positioning a needle guide as described, or claimed, herein, on to the patient over the xiphoid process such that the first indication marker corresponds in position to the xiphoid process; and that a port, of the needle guide, is in the inferior direction from the first indication mark;
- aligning a needle along the trajectory of, or indicated by, a second indication mark of the needle guide; and,
- inserting the needle into the body through the skin towards the desired location.

In some embodiments, the method of guiding a needle comprises using a needle guide as described herein, include wherein a second indication mark, is, or on, or conveys, or indicates, or is configured to convey or indicate, a trajectory of: 217 degrees; or 217 degrees plus or minus 11 degrees; or between 206 degrees and 228 degrees; on the coronal plane, through or to, the port, when measured starting in the sinister direction from the median line, to align the needle.

In some embodiments, the method of guiding a needle comprises the step of: positioning the needle guide over an inferior portion of the xiphoid process of a patient or model, such that the first indication marker corresponds in position to an inferior portion of the xiphoid process; and that a port, of the needle guide, is in an inferior direction from the first indication mark.

In some embodiments, the method of guiding a needle comprises the step of: positioning the needle guide over an inferior portion of the xiphoid process of a patient or model, such that the first indication marker corresponds in position to an inferior portion of the xiphoid process; and that the centre of a port, of the needle guide, is in an inferior direction from the first indication mark, at a distance of 10 millimetres from the first indication mark.

In some embodiments, the method of guiding a needle comprises the step of: positioning the needle guide over the most inferior portion of the xiphoid process of a patient or model, such that the first indication marker corresponds in position to the most inferior portion of the xiphoid process; and that a port, of the needle guide, is in the inferior direction from the first indication mark.

In some embodiments, the method of guiding a needle comprises the step of: positioning the needle guide over the most inferior portion of the xiphoid process of a patient or model, such that the first indication marker corresponds in position to the most inferior portion of the xiphoid process; and that the centre of a port, of the needle guide, is in the inferior direction from the first indication mark, at a distance of 10 millimetres from the first indication mark.

In some embodiments, the method further comprises the step of: positioning the centre of the port in the inferior direction from the first indication mark, above or on, the longitudinal axis of the needle guide, or midline line of the patient or model.

In some embodiment, the centre of the port is positioned to be in the range of 5 to 15 millimetres in the inferior direction from the first indication mark of the needle guide when in normal use. In some embodiment, the centre of the port is positioned to be in the range of 8 to 12 millimetres in the inferior direction from the first indication mark of the needle guide when in normal use.

In some embodiments, the method of guiding a needle comprises guiding a needle for insertion into the heart; or to the heart, for example, to the anterior surface of the heart; or pericardial space of a heart, of a model, or patient. Advantageously the method of guiding a needle may be used for guiding a needle into a model therefore the method can be used for training of surgeons and other medical professionals.

In some embodiments, the desired location is: a heart of a patient; or the surface of the heart, for example the anterior surface of the heart; or a pericardial space of a heart of a patient; or model of a heart, or model of a pericardial space of a heart.

In some embodiments, the method of guiding a needle, for example, for insertion into the pericardial space of the heart of a patient, further comprises the step of: aligning the needle with the trajectory of, or conveyed from, a third indication mark before inserting the needle into the body. Aligning the needle for insertion with the trajectory of a third indication mark gives a greater chance that the needle when inserted will reach the desired location, as the third indication mark may indicate another dimension to the other indication marks.

In some embodiments of a method of guiding a needle, for example, for insertion into the pericardial space of the heart of a patient, further comprises the step of: inserting the needle into the patient in the direction of the desired location, for example, the pericardial space of a heart, by a desired distance. The needle may therefore be positioned easily in the desired position.

In some embodiments, a method of guiding a needle, there is the step of moving the arm of a third indication mark from a first planar configuration to a second configuration wherein the arm of the third indication mark is at least partially perpendicular from the guide body. Advantageously, this may enable the needle guide to be manufactured, transported and stored in a planar configuration, thus taking up less space among other things. Such a feature may enable easier manufacture, transport or storage than a non-planar configuration.

In some embodiments of a method of guiding a needle, there is the step of removing the needle guide, as herein described or claimed, once the needle is positioned for insertion. The removal of the needle guide, when the needle is positioned is made easy, in embodiments that comprise a slot, that the needle can be held in position and the needle guide removed. The slot allows removal of the needle guide, for example, via the slot.

According to another aspect of the present invention there is provided a method of surgery or treatment of a human comprising the method for guiding a needle as herein described, for example, for insertion to the heart, for example, to the anterior surface of the heart or, pericardial space of the heart, of a patient. The present invention may increase the chances of inserting a needle into a patient to the desired location and thus reduce the risk of needless harm to the patient.

According to another aspect of the present invention there is provided a method of training a user to guide a needle, for example, for insertion of a needle to the heart, for example, to the anterior surface of the heart, or pericardial space of the heart, of a patient. The present invention may be used as a training device or method and thus may allow safe training of medical professionals to practice inserting needles and the like. The method of training a user may comprise any feature or step or combination of features or steps as per aspects for a method of guiding a needle, or using a needle guide or the needle guide device, or system thereof, as herein described, or claimed.

According to another aspect of the present invention there is provided a system: comprising a needle guide as herein claimed or described.

In some embodiments, the system further comprises instructions for using the needle guide.

In some embodiments, the instructions for using the needle guide comprise the method steps as described herein to the methods.

In some embodiments, the instructions for using the needle guide as herein claimed or described, comprise the steps of:
- positioning the needle guide, on to the patient over the xiphoid process such that the first indication marker corresponds in position to the xiphoid process; and that a port, of the needle guide, is in the inferior direction from the xiphoid process;
- aligning a needle along the trajectory of the second indication mark;
- and inserting the needle into the body through the skin towards the desired location.

In some embodiments, the instructions for using a needle guide, as herein claimed or described, comprise the steps of:
- positioning the needle guide, on to the patient over the lower portion, for example, the most inferior portion, of the xiphoid process such that the first indication marker corresponds in position to the xiphoid process; and that a port, of the needle guide, is in the inferior direction from the xiphoid process;
- aligning a needle along the trajectory of a second indication mark of the needle guide;
- and inserting the needle into the body through the skin towards the desired location.

In some embodiments, the system further comprises a needle.

In some embodiments, the system further comprises a cannula.

In some embodiments, the system further comprises adhesive. The adhesive may assist in holding the needle guide on the patient.

In some embodiments, the system further comprises a medical delivery device to deliver a medical device, to a desired location, for example, to the heart, for example, the anterior surface of the heart.

### Terms

The term "anterior" as used herein, is used to describe of, on or towards the front portion of the item, or body, when the body is in the standard anatomical position.

The term "central" as used herein is to mean the most central location of an object or shape even when the object or shape is irregularly shaped. Thus includes shaped that are not circular. The skilled person will understand that an exact central position need not be necessary for the invention to work but central enough fit for purpose, and thus this term includes central enough for purpose.

The term "contact surface" as used herein with reference to the guide body is used to refer to the surface that in normal use will contact the patient. The "contact surface" is on the alternative side or opposite side to the "upper surface" of the guide body.

The term "Coronal Plane" as used herein, is used to describe the plane that divides the human body into two, the front or anterior, portion and the back, or posterior portions, and also defines what is also known as the YZ axis. The term is also used to help explain the orientation of items, for example, the needle guide and in particular, the second indication marks as if the needle guide is in the typical orientation and position for use on a patient as if the patient is lying down face upwards, in other words on the coronal plane of a patient or model, is a horizontal plane, per the standard anatomical position of the body. As per the standard anatomical position of a human body for describing orientation and directions.

The term "diameter" as used herein, is used to describe the widest distance across the shape and therefore the shape need not be a circular.

The term "degree" is used to mean a measurement of a plane angle, in which one full rotation is 360 degrees and each part is called a degree.

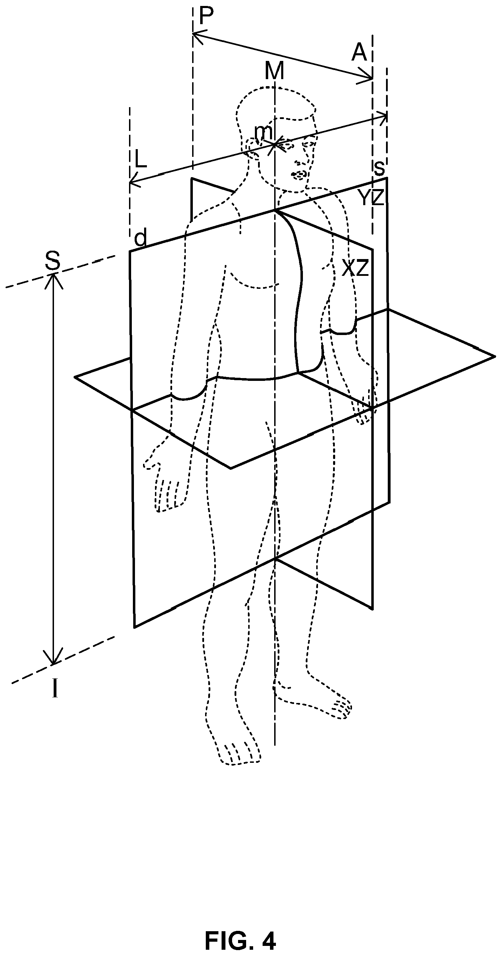

The term "dexter" and the like, as used herein, means of, on, or towards the right hand side, from the bearer's point of view. This is depicted as "d" on Figure 4. Including whether the bearing is a patient, model, human, cadaver or animated object. Unless otherwise specifically stated this term is in relation to the patient or model.

The term "Frontal, or Coronal, Plane" as used herein, is used to describe the plane that divides the human body into two, the front and the back portions, and also defines what is also known as the YZ axis.

The term "hinged" as used herein is used to include foldable and pivotable and the like, and is not necessarily limited to any particular hinged mechanism.

The term "indication mark" as used herein can be any two-dimensional or three-dimensional mark capable of indicating a position, line or trajectory. The indication mark be orientation to be the trajectory line, or be orientated for a part of the trajectory line, and includes conveying a trajectory line.

The term "inferior direction" or "inferior side" as used herein, is used to describe the direction, or side, towards the feet of the body or patient. The term "inferior portion" is used to describe the lower portion of the body, the portion towards the feet. This term includes inferior enough for purpose of working the invention.

The term "length" as used herein, is the distance from end to end of an object or shape. The term includes, the distance which is usually the longest or vertically running distance from one end to another of a shape or object and includes, and is to be taken to be, the longest distance, from one end to another, of a shape or object when the shape or object is irregular in shape. The definition includes that with objects or shapes with two unequal dimensions, the length is the greater of the two.

The term "lumen" as used herein, is used to mean the inner passage of a tube or similar shaped structure, including frustoconical hollow shaped structures.

The term "Median Plane" or "Median (Sagittal) Plane" or "Sagittal Plane" as used herein, is used to describe the plane that divides the human body into two, left and right portions when the body is facing forward, in the head to toe direction.

The "Midline" or "Midline axis" or "median line" runs along the Median Sagittal Plane and is also the XZ axis. Unless otherwise specifically stated these terms are in relation to the patient or model, or the needle guide when orientated in the usual orientation and position in normal use. For example the Median line of the needle guide when in the usual orientation for use, may equate to the longitudinal axis of the needle guide and would be over the median line of the patient or model when in the normal position and orientation for use. These terms are used to aid describing the invention, and an angle that is measured in the sinister direction from the median line, may refer to the median line of the patient, or needle guide, its longitudinal axis, when in the usual position and orientation for use.

The term "model" and "training model" and the like as used herein also includes patients and cadavers for training purposes.

The term, "most inferior portion of the xiphoid process" and the like, is used herein, to mean the most inferior edge of the xiphoid process, in other words the most inferior point of the xiphoid process, which can be detected or located through the skin or by the position of the ribs of the patient or model, when looking face on at the patient or model. The skilled person will understand that the determination of this most inferior point will be exact enough for purpose. The term includes most inferior enough for purpose of working the invention.

The term "needle" as used herein, is used, to describe needles, and cannula and the like.

The term "over the patient", and "over the xiphoid process of the patient, and the like is used herein to mean positioned over the patient, or model, in the anterior direction on the coronal plane, and includes at least partially over. This term also includes aligning, aligning the second indication mark with the xiphoid process, for example aligning the second indication mark with the most inferior portion of the xiphoid process.

The term "patient" as used herein, is used to describe a patient, for example a human, but also a training model, for which the term includes body parts, cadavers, and also animated objects. The known terms to describe a human for example the planes of the human body for example, sagittal, coronal and transverse planes, as well as medial, lateral, inferior and superior direction, for the purposes of describing the invention will apply to the term "patient" regardless if the patient is a human, cadaver or an animated object. The "left" side of the patient will be the patient's left side, including where the patient is a cadaver or an animated object.

The term "posterior" as used herein, is used to describe of, on or towards the back portion of the body when the body is in the standard anatomical position. Unless otherwise specifically stated this term is in relation to the patient or model.

The term "sinister" and the like, as used herein, means of, on, or towards the left hand side, from the bearer's point of view. Including whether the bearing is a patient, model, human, cadaver or animated object. Unless otherwise specifically stated this term is in relation to the patient or model.

The term "superior direction" as used herein, is used to describe of, on, above or towards the upper, head portion of the body or patient, when the body is in the standard anatomical position, or orientation for describing. The term "superior portion" is used to describe the upper or head portion of a body, model or patient.

The term, "standard anatomical position" as used herein, is a standard position of the human body used to help describe directions and orientations of items and parts of the body and in addition as used herein to help describe the invention, and the relationship, direction and orientation of features of the invention to each other and how in use the invention, the needle guide is used. The standard anatomical position of the human body includes where the body is lying horizontal, face up. The term is used to help describe the invention but is not necessarily limiting to that particular orientation of the body, patient or model. Unless specified otherwise the features are described assuming this "standard anatomical position".

The term "trajectory" as used herein, includes the plural of, or more than one trajectory. Usually the trajectory will be planar or straight. The term includes where the trajectory corresponds or aligns with one or more indication marks, or conveyed from one or more indication marks. The term includes possible potential paths, for example, of travel of a needle.

The term "Transverse Plane", is used herein, to describe dividing the body into two, superior (upper) and inferior lower parts, when the body is facing forward. This plane or axis is also known as the XY axis.

The term "upper surface" as used herein with reference to the guide body is used to describe the surface facing away from the patient or model in use, and is the opposite side from the contact surface that faces and makes contact with the patient when in use. The term includes facing in the anterior direction, when the needle guide is orientated in normal use.

The term "width" as used herein, is the distance across a shape or object and includes, and is to be taken to be, the longest distance across a shape or object when the shape or object is irregular in shape. The definition includes that with objects or shapes with two unequal dimensions, the width is the lesser of the two.

The term "xiphoid process" as used herein, also known as the xiphisternum, is used to describe the small cartilaginous extension at the lower (inferior) end of the breastbone or sternum of a body. At birth, it is a typically thin, roughly triangular region of cartilage, which gradually turns to bone in adulthood and forms part of the sternum.

It will be understood that for the ease of explaining the interrelationship and orientation of features these are to be, unless specifically specified, the orientation when in the orientation for normal use, with the patient or model in the standard anatomical position, namely the patient lying horizontal, face upwards, limbs to the side. Also unless specifically stated the orientations are in reference to the patient not the care user, thus for example, the left or sinister direction is the left of the patient or model, not how seen by the care user.

Any one or more of the features, or steps, of any aspect, embodiment or example of the present invention may be combined with any other one or more feature, or step, of any other aspect, embodiment or example of the present invention.

Non-limited examples of the present invention will now be described in detail with reference to the figures:
Figure 1A, shows a top view of an embodiment of the present invention;
Figure 1B shows a perspective view of the embodiment of figure 1A, and positioning on a model or patient;
Figure 1C, shows a top view of another embodiment of the present invention in a first configuration;
Figure 1D, shows a perspective view the embodiment of Figure 1C, in a second configuration, and positioning on a model or patient;
Figure 2A, shows the angle a of the trajectory projected from the second indication mark;
Figure 2B, shows the angle b of the trajectory projected from the third indication mark;
Figure 3A shows a prospective view of an embodiment of the present invention on a body;
Figure 3B shows a perspective view of the embodiment of Figure 3A and needle and trajectory, in relation to a patient or model.
Figure 4 shows a perspective view of a body's relative direction to aid understanding of the present invention.

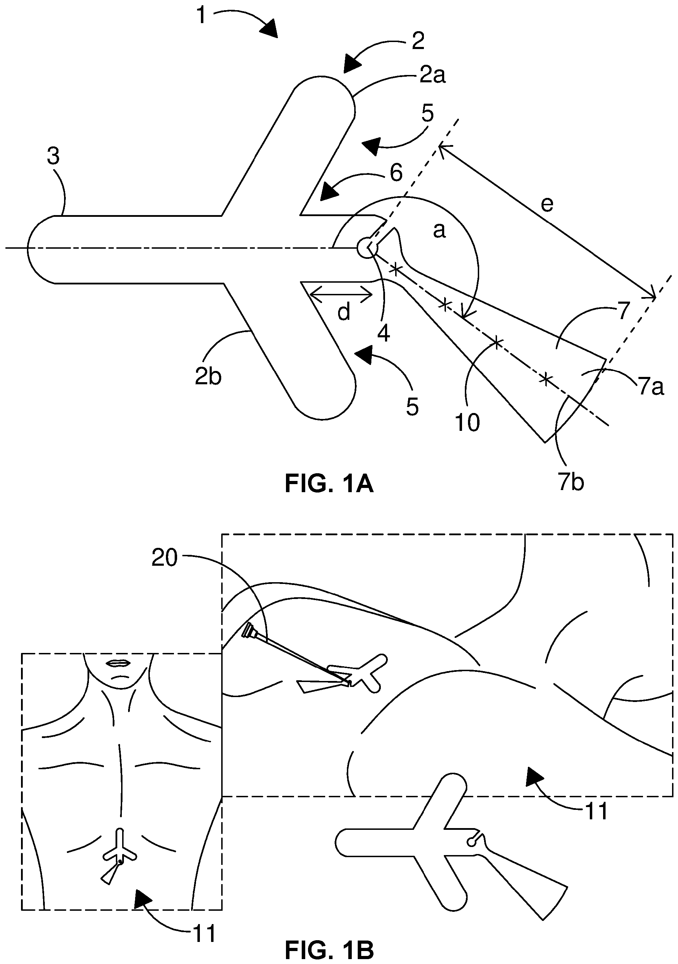

Figure 1A and 1B shows a needle guide 1 of the present invention, comprising a first indication mark 2. The first indication mark 2 comprises two arms 2a and 2b projecting from the same plane as the body 3 of the needle guide 1. In this embodiment, the arms 2a and 2b project from opposite sides of the body 3 of the needle guide 1. In this embodiment, the arms 2a and 2b do not project perpendicular from the body 3 of the needle guide 1 but in other embodiments, one of more of the arms 2a and 2b, may project perpendicularly from the body 3 of the needle guide 1. The arms 2a and 2b in this embodiment are mirror images of each other along the longitudinal axis of the body 3 of the needle guide 1. The arms 2a and 2b both meet the body 3 of the needle guide 1 at the same distance along the longitudinal length of the body 3 but on opposite sides along the longitudinal length of the body 3 of the needle guide 1. The body 3 of the needle guide 1 also comprises a port 4, the needle guide is configured such that, in normal use, the port is at the inferior portion of the needle guide 1. In this embodiment, the port 4 is circular, but also with an opening slot from one side, although in other embodiments the port 4 may be a different shape. In this embodiment, the diameter of the port 4 is 5 millimetres. The centre of the port 4, in this embodiment, is 10 millimetres from the line across the longitudinal length of the body 3 at the inferior side 5 of the two arms 2a and 2b of the first indication mark 2, where the two arms 2a and 2b meet the body 3 of the needle guide 1. In this embodiment, the portion of the first indication mark that should be positioned over the most inferior portion of the xiphoid process is the, most inferior point where the two arms join. In this embodiment, the inferior sides 5 of the two arms 2a and 2b of the first indication mark 2, where these inferior sides 5 meet the body 3, are corners 6, that may be positioned on the body so that these two corners 6 correspond to the inferior end of the xiphoid process of the patient and orientated that the port 4 is at the inferior end. In this embodiment, the centre of the port 4 is positioned 10 millimetres below the first indication mark from the point on the longitudinal axis of the needle guide. When the needle guide 1 is positioned as mentioned above with the corners 6 at the inferior end of the xiphoid process the centre of the port 4 will be a distance d from the port, and in this example, this distance d is 10 millimetres below, or in the inferior direction from the xiphoid process.

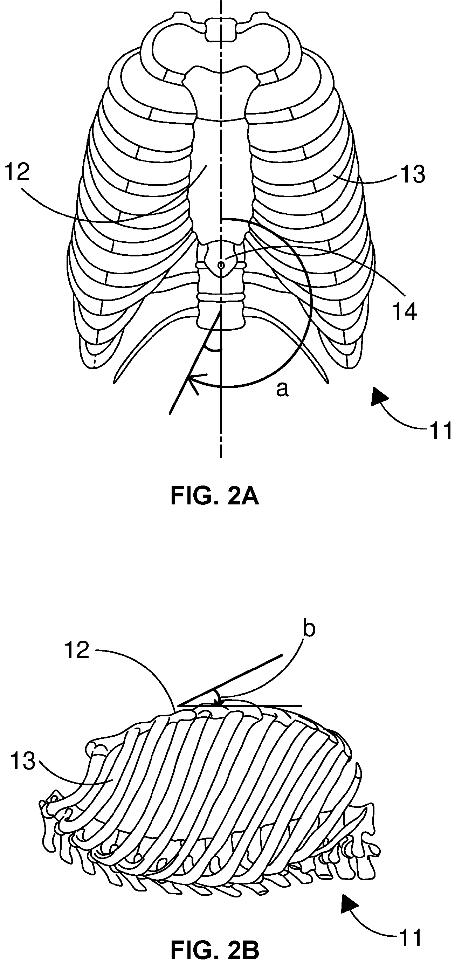

The second indication mark 7, in this embodiment, is an arm 7a that extends from the guide body 3 of the needle guide 1. In this embodiment, the second indication mark 7 extends from the body 3 such that the central longitudinal axis of the second indication mark 7, and as shown by a line 7b, is at an angle of 217 degrees, shown as angle a in figure 1A, from the longitudinal axis (shown as a long-short dashed line for illustrative convenience in Figure 1A) of the needle guide, or median line when in normal use, in an initial sinister direction, on the coronal plane, such that insertion, or movement, of a needle, along the conveyed trajectory of the centre of the second indication mark, in the, lateral to medial direction, through the port will travel in the superior direction on the left, or sinister, side of the patient. In normal use this angle "a" would or could also be measured from the median line of the patient as the longitudinal axis of the needle guide would be aligned with the median line of the patient or model. This is be measured in the sinister direction initially, from the median line. Figure 2A helps show this trajectory projected from the second indication mark 7. Figure 2A shows the xiphoid process 14 of a patient 11, at the inferior end of a sternum 12 and a portion of a rib cage 13. The angle a, is also shown.

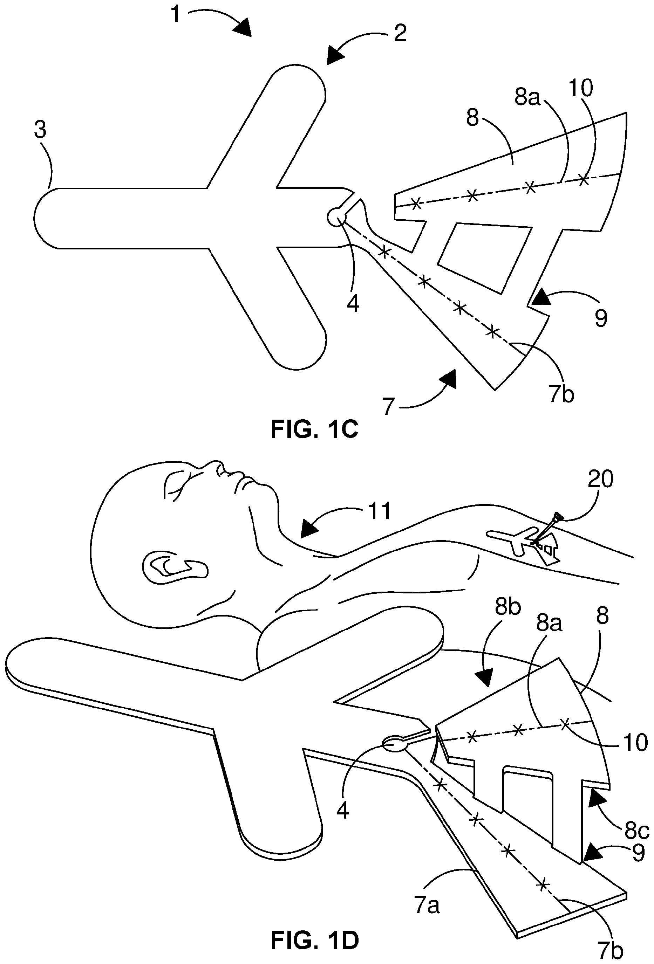

In this embodiment as shown in Figures 1A the line 7b of the second indication mark 7 also comprises distance marks 10 or a fourth indication mark. The distance marks 10 are spaced apart at a defined distance. In this embodiment of 1A the distance marks are spaced apart by 10 millimetres, the drawings are not necessarily drawn to scale. In this Figure 1C embodiment, the line 7b is shown as a dashed line, but in other embodiments the line may be solid, of colour, or a different arrangement of dashes or dots, or any other configuration that may project or convey, a line or trajectory. The distance marks 10 in this figure 1C embodiment are shown as crosses, X, but in other embodiments may be a different configuration, for example a cross line perpendicular to the line 7b of the third indication mark 7.

In this example of Figure 1A and 1B: the guide body 1 is 40 millimetres in longitudinal length and 12 millimetres wide; the arms 2a and 2b of the first indication mark 2 are 25 millimetres in longitudinal length; the second indication mark 7 comprises an arm that is 40 millimetres in the longitudinal length, shown as e in Figure 1A. Figure 1B shows the positioning of needle guide 1 in relation to a patient 11 or model 11, and a point of a needle 20 in relation to the needle guide 1 and patient or model 11, in line with the port 4.

The embodiment of figure 1C and 1D comprises similar features as seen in the Figure 1A and 1B embodiment, for example, the guide body 3, port 4, first indication mark 2 and second indication mark 7. In addition, the figure 1C, 1D embodiment has a third indication mark 8 that is attached to the second indication mark 7 at points 9. The attachment points 9 are, in this example, hinge connections 9, this allows that the device can be manufactured and transported flat in one-dimensional plane. In this example, the hinge 9 is a folding point, that enables the third indication mark 8 to be moved from a first configuration, as shown in Figure 1C, flat or of one plane, to a second configuration, as shown in Figure 1D where the third indication mark is perpendicular to the second indication mark. In other embodiments, the hinge mechanism or feature may comprise other means to enable the movement between the two configurations. There is also every configuration in-between flat and perpendicular. For use, the third indication mark 8 can be pivoted at the hinged points 9 to be perpendicular to the plane of guide body 3 and second indication mark 7. The third indication mark 8 may be retained in this perpendicular position, from the guide body 3. In this example, the third indication mark 8 can be folded at hinged points 9 to move the third indication mark 8 from a flat planar position, planar with the guide body 3 to a perpendicular position from the plane of the guide body 3, and retained at this perpendicular position from the plane of the guide body 3. A person skilled in the art will recognise that an exact perpendicular angle is not required for the invention to work and be effective.

In this example shown and described according to Figure 1C and 1D the third indication mark 8 comprises a line 8a generally along the centre of the longitudinal length of the third indication mark 8. The line 8a, in this example, is on, or travels along, a trajectory, or projects a trajectory, or is configured to project or indicate a trajectory that is 34 degrees from the plane of the guide body 3, and that would meet the centre of the port 4, if so extended, when in the normal orientation for use. Figure 2B helps show this 34-degree angle b from the patient 11, assuming the guide body 3 is flat on the patient 11. In this example, the line 8a is on the upper side of the third indication mark 8 when the third indication mark 8 is planar with the guide body 3. Thus in, this example, when the third indication mark 8 is in the perpendicular orientation, folded to be perpendicular from the plane of the guide body 3, a user can see both the line 8a of the third indication mark 8 and the line 7b of the second indication mark 7. Therefore, in use the user can align the needle with the centre of the port 4; the longitudinal central axis of the second indication mark, the line 7b; and the central longitudinal axis of the third indication, mark 8a, for insertion of a needle 20. Such a trajectory for the insertion of a needle 20 may have a greater chance of reaching the desired location. In this embodiment of Figures 1C and 1D the line 8a of the third indication mark comprises a dashed line, and is shown in the figures to comprise a dashed line. In other embodiments, the line 8a of the third indication mark 8 may be a different configuration, for example, a solid line. The line 8a in other embodiments may comprise a colour.

In this example, the third indication mark 8 comprises a fourth indication mark 10 which comprises a series of distance marks 10 along the length of the line 8a, these marks 10 are 10 millimetres distanced from one another along the length of the line 8b, in this embodiment of Figure 1C and 1D shown, although the figures may not necessarily be drawn to scale. In this embodiment of figure 1C and 1D the distance marks are seen as crosses but the distance marks, fourth indication marks 10 may be other configurations that are able to convey distance along the third indication mark 8. When the needle is aligned with one of these distance marks 10 it is therefore easy to calculate how far the needle 20 has been inserted into the patient 11, even when the tip of the needle 20 is out-of-sign for example, below the skin of the patient 11.

In this example, of Figure 1C and 1D, the upper edge 8b of the third indication mark 8 travels along a trajectory that when the third indication mark is perpendicular to the plane of the guide body, the trajectory of the upper edge 8b of the third indication mark 8 is at 46 degrees from the plane of the guide body 3. Ideally, and also in this example, the trajectory of the upper edge is to the centre of the port 4.

In this example of Figure 1C and 1D, the lower edge 8c of the third indication mark 8 travels along a trajectory that when the third indication mark is perpendicular to the plane of the guide body, the trajectory of the lower edge 8c of the third indication mark 8 is at 22 degrees from the plane of the guide body 3. Ideally and also in this example, the trajectory of the lower edge is to the centre of the port 4.

In this example of Figure 1C and 1D the longitudinal length of the third indication mark 8 is 40 millimetres in length. The width of the third indication mark 8 at the longest section is 35 millimetres in length. The width of the third indication mark 8 at the shortest width is 10 millimetres in length. The port 4 is 5 millimetres in diameter; the longitudinal length of the guide body 3 is 45 millimetres in length and 12 millimetres in width. The longitudinal length of the arm of the second indication mark is 40 millimetres.

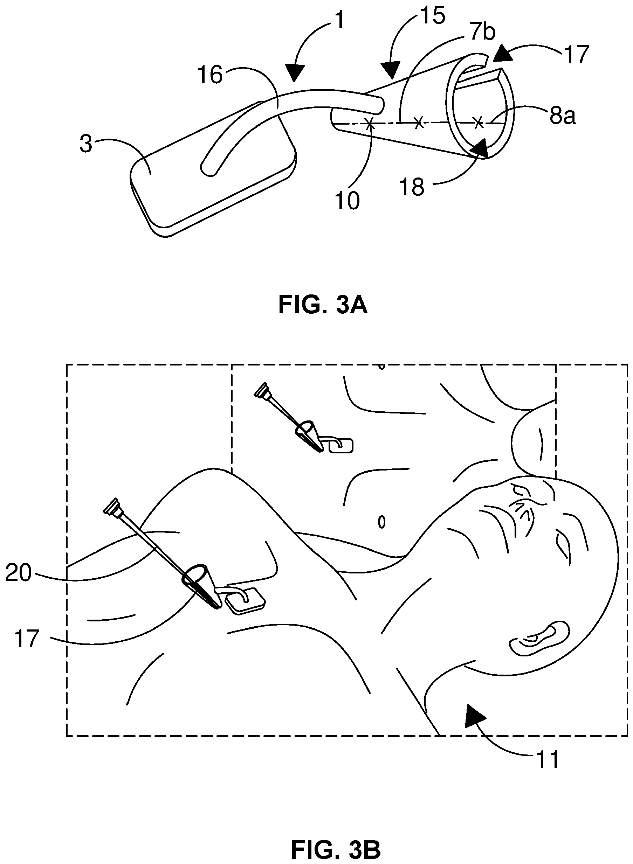

Figure 3A and figures 3B are to another embodiment of the present invention. In this figure 3A, 3B example the guide body 3 is rectangular shape and in this example is 30 millimetres in length and 20 millimetres in width. The guide body 3 comprises a contact side (not in view) to make contact with a patient 11 when in use and to fix the guide body 3 onto the patient 11. In this example, the second 7 and third 8 indication marks are merged into a rigid, generally tubular, funnel shape element 15. In this example, the second 7 and third 8 indication marks comprise the same feature or of the same feature - the funnel shaped element 15. The funnel shaped element 15, is positioned and held from the guide base 3 by a rigid extension 16. In other embodiments a funnel shaped element 15, or second 7 and third 8 indication marks may be orientated from the guide body 3 by other means, for example, an end of the funnel shaped element 15, or second 7 and third 8 indication marks may be joined directly to the guide body 3. The rigid extension 16 holds the funnel shaped element 15 at the desired angle or angles. The funnel shaped element 15 comprises a slot 17, that allows easy access, and exit, of a needle 20 by a user into the enclosed space 18 of the funnel shaped element 15. After positioning of the needle, the slot 17 is also advantageous for allowing easy removal of the needle 20 guide while keeping the needle 20 in position. The slot 17, in this example, opens from the larger opening of the funnel shaped element 15, at the end of the funnel shaped element 15 furthest from the patient 11 when in use. In this example, the port 4 is the smaller opening of the funnel shaped element 15 nearer to the patient 11 when in use than the larger opening of the funnel shaped element 15. In this example, the funnel shaped element 15 has lines 7b and 8a on the outer and inner walls of the funnel shaped element 15. In this embodiment although lines 7b and 8a are on either the inner or the outer surface of the funnel shaped element 15 they are aligned in view. As with the Figure 1 examples, the lines 7b and 8a conveys or projects a trajectory suitable for inserting a needle into the patient 11 to reach a desired location. In this example, the lines 7a 8a also comprise distance marks 10 space apart 5 millimetres apart from the next distance mark 10.

In use, the side of the guide base 3 that is nearest to the funnel shape element 15 is positioned at the inferior edge of the xiphoid process, and is the first indication mark 2. When the needle guide 1 is in this position the funnel shaped element 15 is held by the rigid extension 16 so that the centre axis of the funnel shaped element 15 conveys, or is, one trajectory that will meet with the patient 11 ten millimetres from the edge of the guide body 3 in the inferior direction from the guide body 3, and the axis of the funnel shaped element 15 is 34 degrees from the plan of the guide body 3 and is 217 degrees from the median line of the patient 11.

The funnel shaped element 15 is 40 millimetres in longitudinal length, in this example, although other examples may have different lengths.

All ranges include the maximum and minimum points disclosed and include any intermediate ranges therein, which, may or may not, be specifically enumerated herein. Within this context, a number may be considered, to include numerical values that are general standard error for the measurement of the property that the number modifies.

Figure 4 shows various reference points of a patient or model, for example the median line M, or longitudinal axis of the patient. The median line M runs in the superior S (upper) to inferior I (lower) direction and vice versa. The anterior A and posterior P directions are also shown. The sinister s (left) and dexter (right) d directions are also shown. The coronal YZ plane (also known as frontal plane) and the sagittal XZ plane is also shown. As well as the lateral L direction and the medial m directions.

## Claims

1. A needle guide, for positioning of a needle or the like, wherein the needle guide comprises:
- a guide body, wherein the guide body comprises: a lower contact surface and an upper surface wherein the lower contact surface is configured for positioning, on a patient, at least partially over the xiphoid process of the patient;
- a port;
- a first indication mark configured to be positioned over an inferior portion of the xiphoid process, with an orientation that the port of the needle guide is in an inferior position to the first indication mark, such that when in use, the first indication mark indicates the position of the inferior portion of the xiphoid process of the patient and the port of the needle guide is inferior in position to the first indication mark; and,
- a second indication mark that indicates, or is configured to indicate, a trajectory of: 217 degrees; or 217 degrees plus or minus 11 degrees; or between 206 degrees and 228 degrees; from the median line, on the coronal plane, through or to, the port, when measured starting in the sinister direction from the median line.

2. A needle guide as claimed in claim 1 wherein the first indication mark comprises, at least one arm that extends from the guide body in the same plane as the guide body.

3. A needle guide as claimed in any preceding claim where the second indication mark comprises an arm that extends from the guide body in the same plane as the guide body.

4. A needle guide as claimed in any preceding claim wherein the port is circular.

5. A needle guide as claimed in any preceding claim wherein the second indication mark is, or is at, or is on, or conveys, or indicates, or is configured to indicate, a trajectory that goes through, or to, the centre of the port.

6. A needle guide as claimed in any preceding claim wherein the needle guide further comprises a third indication mark, that comprises an arm extending from the guide body, wherein the arm is capable of being orientated perpendicular from the upper surface of the guide body, away in direction from the patient.

7. A needle guide as claimed in claim 6, wherein the third indication mark is, or is at, or is on, or conveys, or indicates, or is configured to indicate, a trajectory of: 34 degrees; or 34 degrees plus or minus 12 degrees; or is between 22 degrees and 46 degrees, from the coronal plane through, or to, the port.

8. A needle guide as claimed in claim 6 or 7, wherein the third indication mark conveys or indicates a trajectory through, or to, the centre of the port.

9. A needle guide as claimed in claim 6, 7 or 8, wherein the third indication mark comprises a distal edge that projects, or indicates, the trajectory of: 34 degrees; or 34 degrees plus or minus 12 degrees or is between 12 degrees; and 34 degrees, from the Frontal plane through, or to, the port.

10. A needle guide as claimed in any one of claims 6 to 9, wherein the third indication mark comprises a visual mark which indicates the trajectory of: in the range of 22 degrees to 46 degrees, from the coronal plane through, or to, the port.

11. A needle guide as claimed in any one of claims 6 to 10 wherein the third indication mark comprises a tubular shape.

12. A needle guide as claimed in claim 6 to 11 wherein the third indication mark comprises a frustoconical shape, with the smaller diameter opening nearer to the port than the larger diameter opening.

13. A needle guide as claimed in any one of the claims 6 to 12 wherein the third indication mark comprises a slot.

14. A needle guide as claimed in any one of the preceding claims further comprising wherein the centre of the port is configured 10 millimetres in the inferior direction of the needle guide, when in normal use, from the first indication mark.

15. A needle guide as claimed in any preceding claim wherein the needle guide comprises a fourth indication mark configured to indicate a defined distance from the port along a trajectory.
